# EUROPEAN PATENT SPECIFICATION

(11) **EP 3 897 342 B1**
(45) Date of publication and mention of the grant of the patent: **15.11.2023**
(21) Application number: 19899925.2
(22) Date of filing: 08.12.2019
(51) Int. Cl.: A61B 1/00, A61B 90/50, A61B 1/313, A61B 90/00

(54) **ENDOSCOPE/LAPAROSCOPE EASY ATTACH-DETACH MECHANISM**
EINFACHER BEFESTIGUNGS-/LÖSUNGSMECHANISMUS FÜR EIN ENDOSKOP/LAPAROSKOP
MÉCANISME D'ATTACHEMENT/DÉTACHEMENT FACILE D'ENDOSCOPE/LAPAROSCOPE

(30) Priority: 18.12.2018 TR 201819682
(43) Date of publication of application: 27.10.2021
(73) Proprietor: IZMIR YÜKSEK TEKNOLOJI ENSTITÜSÜ, 35430 Izmir (TR)
(72) Inventor: DEDE, Mehmet Ismet Can, Mühendisligi Bölümü Gülbahçe, Izmir (TR); MOBEDI, Emir, Mühendisligi Bölümü, Urla/Izmir (TR)
(86) International application number: PCT/TR2019/051043
(87) International publication number: WO 2020/130977

(56) References cited:
- JP-A- 2000 316 794
- KR-A- 20180 044 627
- US-A- 5 571 072
- US-A- 5 571 072
- US-A1- 2009 026 682

## Description

### Technological Field:

The invention is related to endoscope/laparoscope directing robot systems and endoscope/laparoscope holder systems used in the minimally invasive surgery. The present invention is related to a mechanism which allows for holding the endoscope/laparoscope which is a camera system used in the minimally invasive surgical systems, by means of the robot or an endoscope/laparoscope holder and when necessary, allows for detaching the endoscope/laparoscope easily from the holder and rotating around the telescope axis with a limited angle.

### State of the Art:

The endoscope/laparoscope holder systems are divided into two as active and passive in the literature. In order to change the position of the endoscope/laparoscope attached onto the tip portion of the passive holders, adjustable friction or pneumatic systems are used in the joints of the holder in order to fix the direction of the endoscope/laparoscope. The operator fastens the endoscope/laparoscope to the holder mechanically in the beginning of the surgery. The position of the endoscope/laparoscope is ten changed by manipulating the passive holder joints manually by the operator. In active holders, the movement of the endoscope/laparoscope is achieved by means of the actuators located on the joints of the endoscope/laparoscope holder (it is mostly a robotic system). In the beginning of the operation, the surgeon fastens the endoscope/laparoscope to the tip potion of the endoscope/laparoscope holder robot and the required changes in the position of the endoscope/laparoscope is achieved by means of sending drive inputs to the motors on the joints of the active holder (endoscope/laparoscope holder robot).

In both types of endoscope/laparoscope holders, active and passive holders, the endoscope/laparoscope is fastened onto the holder however this fastening process is performed by means of the surgeon using both hands simultaneously. In this case, the surgeon shall pause the surgery and shall deal with this attaching or detaching process of the endoscope/laparoscope. Also, the conditions in different areas of the surgical region are examined by means of using the angled telescopes of the endoscope/laparoscopes. In this case the endoscope/laparoscope shall be rotated around the telescope axis. In the present design of active and passive endoscope/laparoscope holders, since the endoscope/laparoscope is fastened, first of all, it shall be detached and then rotated and then attached again.

In the patent document No US6569084 B1 encountered during the literature survey, the endoscope/laparoscope is fastened to the endoscope/laparoscope holder. The portion named as the endoscope/laparoscope holder consists of a passive mechanism. When the operator requires changing the position of the endoscope/laparoscope, he/she brings said holder to the required position by means of changing the friction of the joints of the holder in a manual manner. In this patent, there is no invention concerning easy attaching and detaching of the endoscope/laparoscope.

In another patent document No US 5571072 encountered during the literature survey, holding the elements such as the endoscope and laparoscope used in robotic surgical applications by means of a passive holder independent from the doctor during the surgery is disclosed. The endoscope/laparoscope is fixed by means of the designed apparatus with the help of a clip under the effect of the frictional force. When it is required to change the position of the endoscope/laparoscope, the clip is opened and the position is changed. In this patent, there is no invention concerning easy attaching and detaching of the endoscope/laparoscope.

In the patent document No JP2018068517 (A), a new holder is disclosed with the capability to attach and detach endoscope/laparoscope relatively more easily with respect to the prior ones even when it is inserted into the body. In this patent there is no invention concerning easy attaching and detaching of the endoscope/laparoscope.

Another passive holder example is the endoscope/laparoscope holder system named as Endoworld^{®}LAP53 manufactured by the KarlStorz Company. In said system, the endoscope/laparoscope is fastened to the holder robot through the telescope portion by means of the clip at the beginning of the surgery. In this patent there is no invention concerning easy attaching and detaching of the endoscope/laparoscope.

The camera system inserted in the body during the minimally invasive surgeries becomes dirty in time. In this case, generally, the camera system is removed from the surgical area and cleaned, and reinserted again. In cases where the priory mentioned active or passive holder is used, removing the endoscope/laparoscope out of its fastened location on the holder in order to clean its tip takes too much time. In addition to this, angled endoscope/laparoscope telescopes are used in order to monitor the regions of the surgical area which are difficult to reach. In this case, the endoscope/laparoscope is required to be rotated around the telescope axis.

As a result, an endoscope/laparoscope easy attach-detach mechanism is required wherein the present state of the art is exceeded, disadvantages are eliminated.

### Brief Description of the Invention:

The invention is defined by claim 1 and relates to an endoscope/laparoscope easy attach-detach mechanism which exceeds the present state of the art, eliminates the disadvantages, and brings some extra advantages.

The aim of the invention is to provide a novel easy attach-detach mechanism which enables attaching the endoscope/laparoscope by means of using one hand of the surgeon onto the robot holder or the passive holder before the surgery and/or during the surgery.

Another aim of the invention is to provide a novel easy attach-detach mechanism which allows for rotating the endoscope/laparoscope around the telescope axis within a predetermined angle limit by the surgeon by using his/her one hand while the endoscope/laparoscope is attached onto the robot holder or the passive holder.

Another aim of the invention is to provide a novel easy attach-detach mechanism which allows for detaching the endoscope/laparoscope by the surgeon by using one hand from the robot holder or the passive holder during the surgery when the endoscope/laparoscope is required to be removed.

In order to fulfill all aims mentioned above and emerged out of the following detailed description, the present invention is a mechanism which allows for holding the endoscope/laparoscope, which is a camera system used in the minimally invasive surgical systems, by means of a robot or a passive holder and when necessary allows for detaching the endoscope/laparoscope easily from a robot or passive holder and rotating around its telescope axis with a limited angle, characterized in that; it comprises a fragmented fixed intermediate adaptor which is mounted on the external surface of the endoscope/laparoscope and is engaged with the main chassis in the linear locking mechanism, a return spring which is attached to a main chassis on one end and is attached to a pressing arm on the other end and applies upward pulling force to the cam press arm, a pressing arm which transmits the upward force coming from the return spring and the downward force coming from the surgeon to the compression flange, a compression flange which transmits the upward movement resulting from the return spring's force and the downward movement resulting from surgeon's pressing force onto the pressing arm to the movable flange by means of the connecting pins, a movable flange which compresses the fragmental fixed intermediate adaptor to the main chassis that has contact with it and prevents its motion when a force arises from the return spring, and then allows the fragmental fixed intermediate adaptor to rotate around the endoscope/laparoscope telescope axis by releasing the compression forces on it when the compression force arises from the surgeon's pressing force, a barrier pin which is pushed out when the movable flange moves downwards until the pressing arm is moved to the stopper pin by the surgeon as he/she pushes onto the pressing arm and as a result of this pushes the fragmental fixed intermediate adaptor and thus the endoscope/laparoscope out of the main chassis, a compression spring which is located behind said barrier pin and enables the required force for the barrier pin to push the fragmental fixed intermediate adaptor out of the main chassis.

Another characteristic of the invention is that; it comprises a plurality of connecting pins which are located between the compression flange and the movable flange and transmits the motion coming from the compression flange to the movable flange.

Another characteristic of the invention is that; it comprises springs as many as the connecting pins which are located on the connecting pins between the compression flange and the movable flange and drives the movable flange in the upward direction.

Another characteristic of the invention is that; it comprises an upper stopper pin located on the main chassis which limits the upward movement of the pressing arm by means of the drive coming from the return spring.

Another characteristic of the invention is that; it comprises a lower stopper pin located on the main chassis which limits the downward movement of the pressing arm as a result of the pressure force resulting from the surgeon's pressing force.

Another characteristic of the invention is that; it comprises an adaptor centering pin which is used for centering during the assembly of the two intermediate adaptors mounted on the endoscope/laparoscope.

Another characteristic of the invention is that; it comprises adjusting screws which fasten the fixed intermediate adaptors to the endoscope/laparoscope.

Another characteristic of the invention is that; it comprises the driving parts which are located in the inner surface of the adaptor in order to limit the relative movement of the endoscope/laparoscope in the fragmental fixed intermediate adaptor.

Another characteristic of the invention is that; it comprises a mounting interface which is mounted by means of manually opened/closed fasteners after being centered by means of the centering pins to the chassis of the linear locking mechanism.

Another characteristic of the invention is that; it comprises an angular position sensor which is fastened to the mounting interface, is engaged with the angular position sensor transmitter located on the main chassis and transmits the angular motion data to the measurement systems.

Another characteristic of the invention is that; it comprises a bearing which allows the mounting of the pressing arm on the main chassis.

Another characteristic of the invention; it comprises a compression spring adjusting screw which adjusts the compression force of the compression spring located behind the barrier pin.

### Description of the Figures:

The invention will now be described with reference to the attached drawings, thus the characteristics of the invention will be understood more clearly. However, the aim of this is not intended to limit the invention with possible embodiments. On the contrary, the invention aims to cover all alternatives, amendments and equivalences which can be included within the area defined by the attached claims. Shown details are only for the purpose of describing the preferred embodiments of the present invention and for illustrating the methods and for clarifying the rules and conceptual characteristics of the present invention in a useful and easily understandable manner. In these drawings;
- Figure - 1: is a perspective view of the inventive easy attach-detach mechanism.
- Figure - 2: is an exploded view of the connection of the fixed intermediate adaptor of the endoscope/laparoscope.
- Figure - 3: is an exploded view of the linear locking mechanism.
- Figure - 4: is an exploded view of the connection of the linear locking mechanism to the mounting interface.
- Figure - 5: is a perspective view of the condition in which the endoscope/laparoscope is locked.
- Figure - 6A: is a front view showing the locked condition of the endoscope/laparoscope.
- Figure - 6B: is a section A view.
- Figure - 6C: is a detailed section B view.
- Figure - 7: is a perspective showing the condition where the rotation of the endoscope/laparoscope around the telescope axis is permitted.
- Figure - 8A: is a front view showing the condition where the rotation of the endoscope/laparoscope around the telescope axis is permitted.
- Figure - 8B: is a section C view.
- Figure - 8C: is a detailed section D view.
- Figure - 9: is a perspective view of the condition where the endoscope/laparoscope is in free to be removed status.
- Figure - 10A: is a front view of the condition where the endoscope/laparoscope is in free to be removed status.
- Figure - 10B: is a detailed section E view.
- Figure - 10C: is a detailed section F view.

The figures clarifying this invention are enumerated as it is shown in the attached drawing and their names are listed below.

### Description of References:

**1.** Easy attach-detach mechanism
**10.** Endoscope/laparoscope
**11.** Adaptor centering pin
**12.** Fragmental fixed intermediate adaptor
**13.** Driving part
**14.** Adjusting screw
**20.** Mounting interface
**21.** Manually opened/closed fastener
**22.** Angular position sensor connecting screw
**23.** Centering pin
**24.** Angular position sensor
**30.** Locking mechanism
**31.** Main chassis
**32.** Movable flange
**33.** Connecting pin
**34.** Bearing
**35.** Pressing arm
**36.** Angular position sensor magnet (if a magnetic encoder is used)
**37.** Set-screw
**38.** Compression flange
**39.** Driving spring
**40.** Return spring
**41.** Barrier pin
**42.** Compression spring
**43.** Compression spring adjusting screw
**44.** Upper stopper pin
**45.** Lower stopper pin

### Description of the Invention:

In this detailed description, the inventive easy attach-detach mechanism (1) is described by means of the examples only for clarifying the subject matter, in a manner such that no limiting effect is created. In the description, an easy attach-detach mechanism (1) which allows for holding the endoscope/laparoscope (10) which is a camera system used in the minimal invasive surgical systems by means of the robot or a passive holder and when necessary allows for detaching the endoscope/laparoscope (10) easily from the holder and rotating around the telescope axis with a limited angle is disclosed.

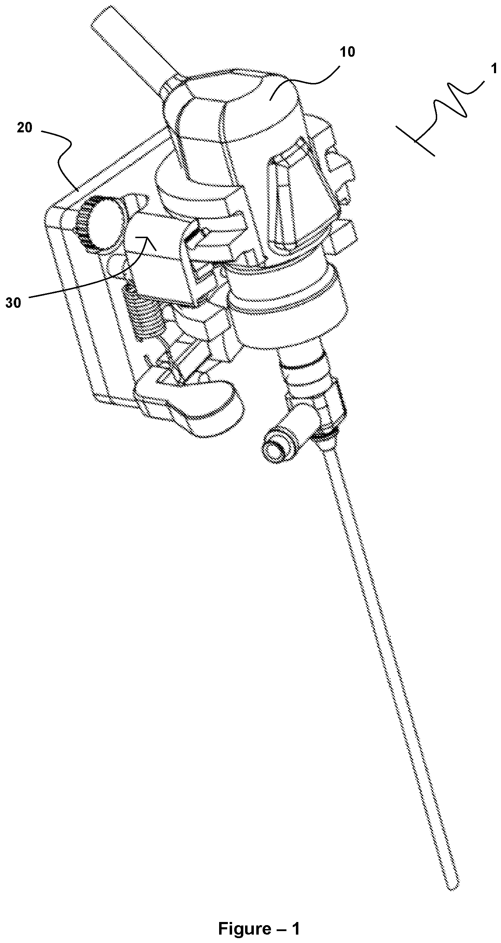

In Figure 1, a perspective view of the inventive easy attach-detach mechanism (1) is given. According to the figure, the invention comprises a mounting interface (20) and a locking mechanism (30) in order to perform the holding process of endoscope/laparoscope (10). The locking mechanism (30) allows for the surgeon to realize a limited angular rotational motion around the telescope of the endoscope/laparoscope (10) by using his/her one hand and for performing endoscope/laparoscope attaching-detaching motion. The mounting interface (20) is used for connecting the easy attach-detach mechanism to the holder. Also only in the section on the mounting interface, there is an electronic component, the angular position sensor (24). By this means, the locking mechanism (30) can be sterilized independent from the mounting interface (20). A surgery bag is worn on the mounting interface (20) in order to provide sterilization during surgery.

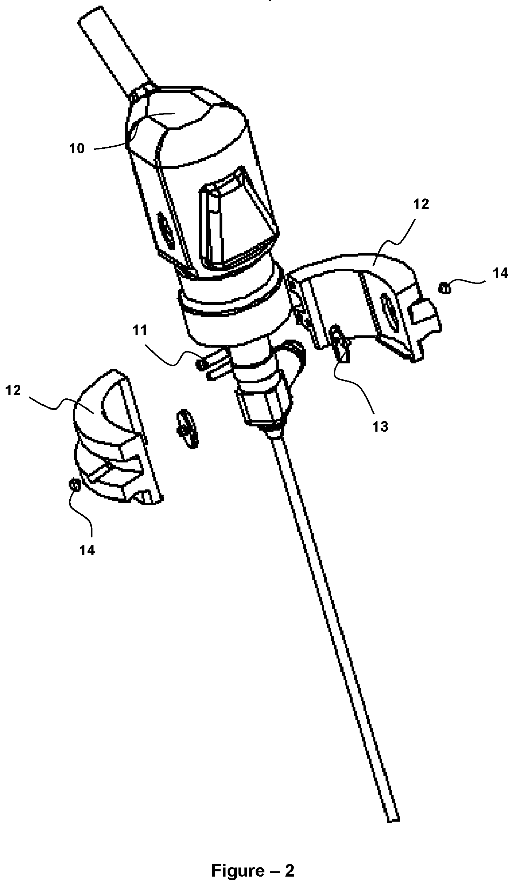

In Figure 2, an exploded view of the connection of the endoscope/laparoscope (10) to the fixed intermediate adaptor (12) is presented. In this figure, there are 2 adaptor centering pins (11), 2 fragmental fixed intermediate adaptors (12) and an endoscope/laparoscope (10), 2 driving parts (13) and 2 adjusting screws (14). During assembly, primarily, the adaptor centering pins (11) are fitted firmly to the fragmental fixed intermediate adaptors (12) which are designed appropriate to the shape of the endoscope/laparoscope (10). Then, 2 fragmental fixed intermediate adaptors (12) are combined by means of the adaptor centering pins (11) and screws (14), one for each.

The driving parts (13) are placed within the slots with an elliptical shape which are located in the inner section of said fragmental fixed intermediate adaptor (12). Then, the adjusting screws (14) are attached through the screw cut holes located on side sections of the fragmental fixed intermediate adaptor (12). These adjusting screws (14) apply pressure to the driving part (13) and thus, prevent the relative motion of the endoscope/laparoscope (10) with respect to the fixed intermediate adaptor (12). The fixed intermediate adaptor (12) is the element which is in contact and in relation with the locking mechanism (30) of the endoscope/laparoscope (10).

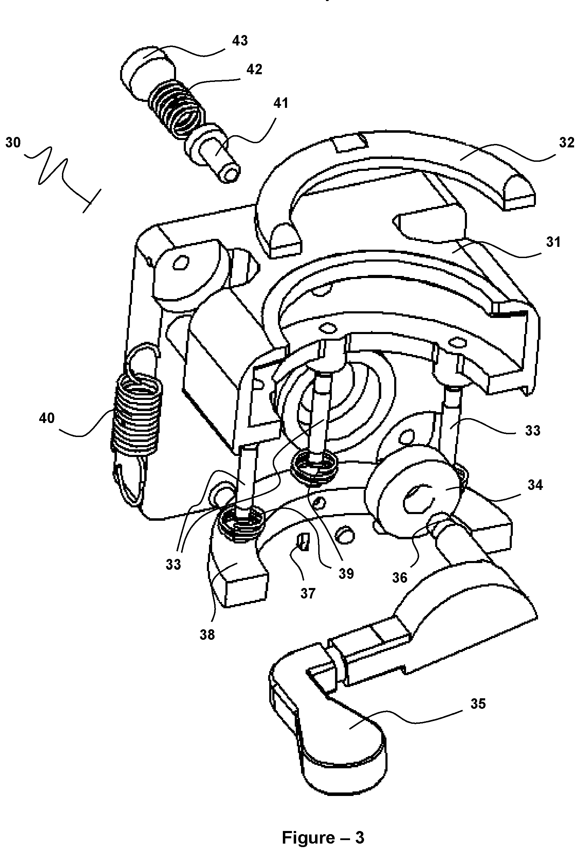

In Figure 3, an exploded view of the locking mechanism (30) is given. According to the figure, there are 1 movable flange (32), 3 connecting pins (33), 3 driving springs (39), 1 compression flange (38), 3 set-screws (37), 1 main chassis (31), 1 bearing (ball bearing) (34), 1 pressing arm (35), a return spring (40), a barrier pin (41), a compression spring (42) and a compression spring adjusting screw (43). The assembly order is as the following; the bearing (34) is attached to the slot in the main chassis (31), the connecting pins (33) are fitted in the slots opened for housing the pins (33) in the main chassis (31), and said pins (33) are screwed into the holes opened on the surface of the movable flange (32). Then, a driving pin (39) is attached under each connecting pin (33) and under it a compression flange (38) is mounted. The set-screw (37) is screwed into the holes opened on the side surfaces of the flange (38) in order to screw the connecting pin (33) via the compression flange (38). Subsequently, the pressing arm (35) is fitted to the bearing (34). By means of said driving springs (39), a continuous contact of the compression flange (38) and the cam pressing button (35) is enabled. The return spring (40) is attached in the slot designed appropriate to the return spring (40) which is located on the cam pressing button (35). Consequently, the barrier pin (41) is attached to the main chassis (31) and then the compression spring (42) and compression spring adjusting screw (43) are mounted. In the locking mechanism (30), there is an angular position sensor magnet (36) (if a magnetic measurement system is used) which is engaged with the angular position sensor (24) on the end of the pressing arm (35).

In the invention, the fixed intermediate adaptor (12), on which the endoscope/laparoscope (10) is attached, is mounted to the locking mechanism (30). When the fixed intermediate adaptor (12) is fitted into the main chassis (31), it is compressed to the movable flange (32) and the main chassis (31) from the bottom portion. By these means, the adaptor (12) cannot move relative to the main chassis (31). The compression force on the movable flange (32) originates from the return spring (40). The return spring (40) pulls the pressing arm (35) upwards and thus, pushes the movable flange (32) to which it is connected by means of the compression flange (38) and pins (33) in the upward direction. In this case, the movable flange (32) fixes the adaptor (12) to the chassis (31) by pushing onto it. The locking process occurs as a result of the linear motion. Under ideal conditions, the mechanism (1) operates in two conditions namely open or closed conditions. At the point of the linear motion creation, cam mechanism on the pressing arm (35) is utilized. The compression flange (38) is kept under continuous contact by means of the pressing arm (35) and the driving springs (39). Since the movable flange (32) is combined with compression flange (38), with the help of the connecting pins (33), each movement realized by the compression flange (38) can be observed in the movable flange (32). When it is pressed on the pressing arm (35), the compression flange (38) enables the downward motion of the movable flange (32) in the downward direction along the connecting pins (33). When the movable flange (32) moves in the downward direction, the fixed intermediate adaptor (12) is released from the compressed situation within the chassis (31). In this case, the fragmental fixed intermediate adaptor (12) fixed on the endoscope/laparoscope (10) is pushed by means of the barrier pin (41) and is removed away from the main chassis (31). The required drive for the pushing motion of the barrier pin (41) originates from the pre-compression of the compression spring (42) which is located behind it.

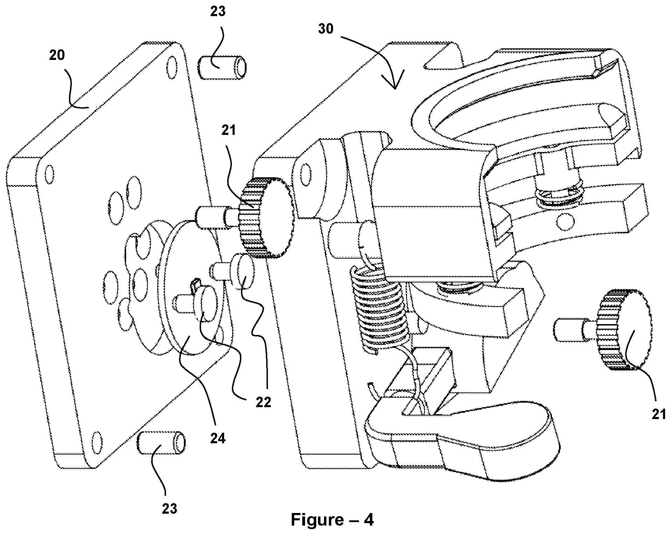

In Figure 4, the assembly condition of the locking mechanism (30) included in the invention with the mounting interface (20) is given. In this figure, there are 2 manually opened/closed fasteners (21) and a centering pin (23), an angular position sensor (24), 2 angular position sensor connecting screws (22), 1 mounting interface (20) and a locking mechanism (30). The locking mechanism (30) is mounted on the mounting interface (20) by means of the centering pins (23) through the centering holes located at the corners of the main chassis (31) and the manually opened/closed fasteners (21) are screwed by hand without using any hand tool on the screw holes at the corners. There is an angular position sensor (24) located on the middle portion of the mounting interface (20) for transmitting the states of the endoscope/laparoscope (10) with respect to the chassis to the measurement systems. The angular position sensor (24) is mounted on the mounting interface (20) by means of the angular position sensor connecting screws (22), and it is in relation with the angular position sensor magnet (36) which is located in the locking mechanism (30).

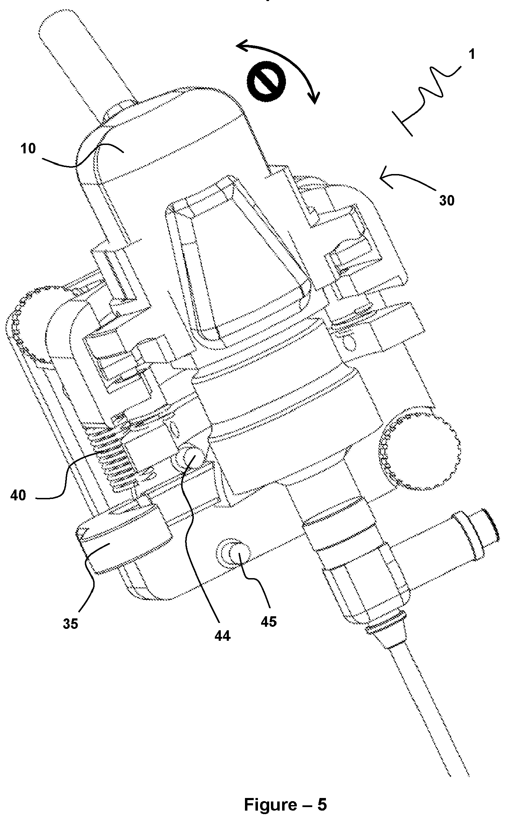

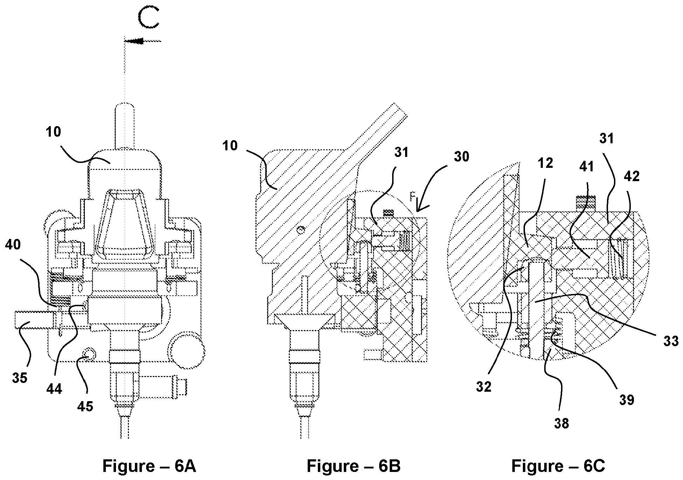

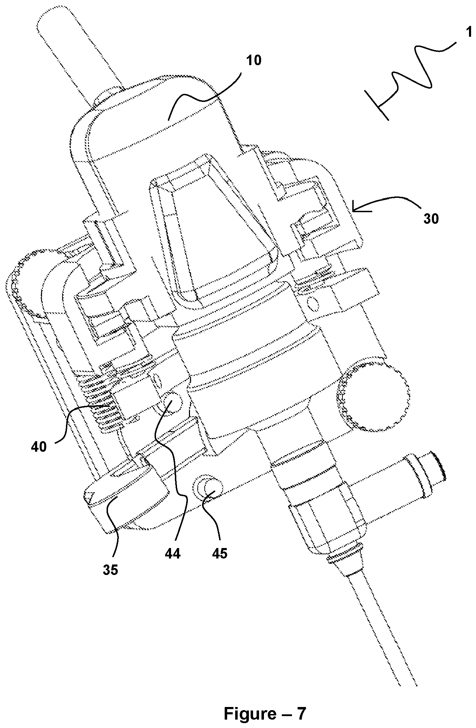

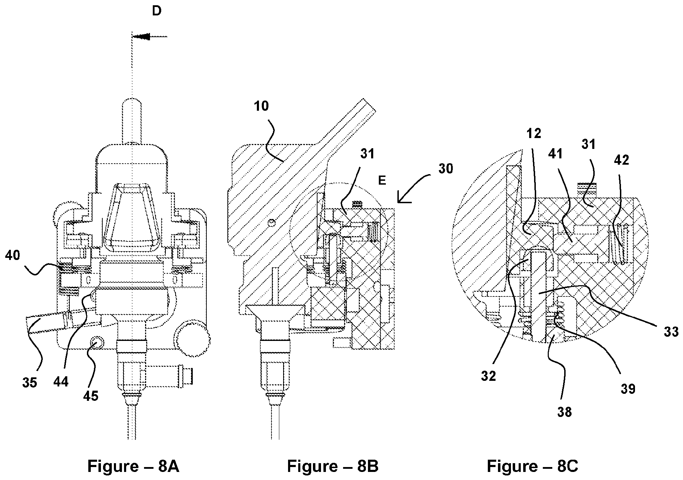

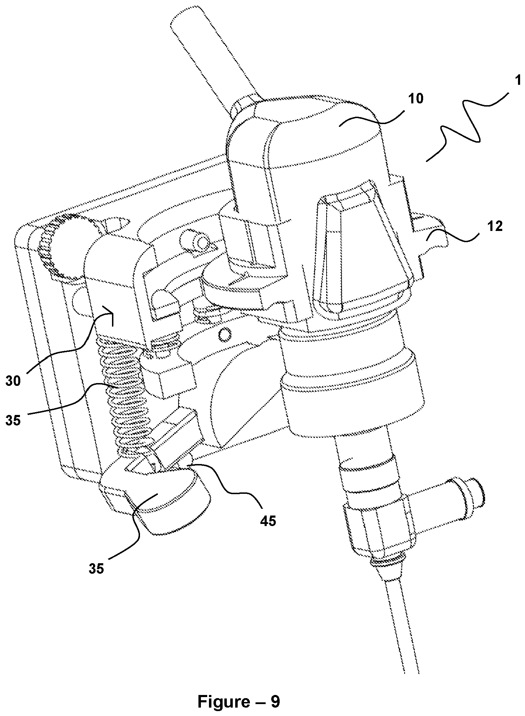

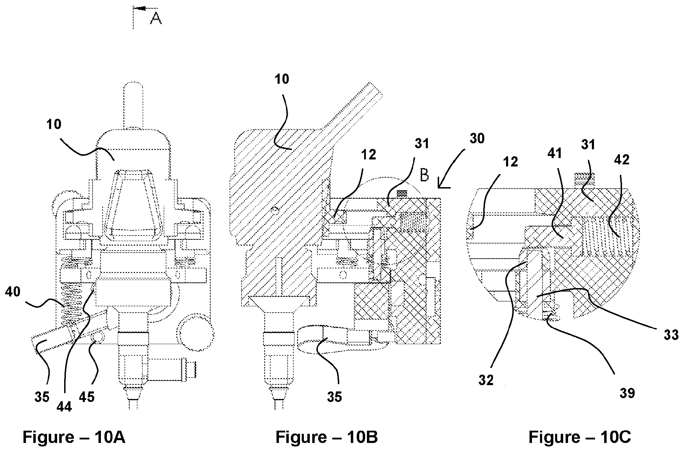

In Figure 5, a perspective view of the endoscope/laparoscope (10) which is locked in the easy attach-detach mechanism (1) is given. In this case, the endoscope/laparoscope cannot move away from the easy attach-detach mechanism (1) and cannot make a rotational motion. In Figure 6A, a front view showing the locked condition is shown, in Figure 6B, the view of the A cross-section is shown, in Figure 6C, the view of the B cross-section is given. In this case, the fragmental fixed intermediate adaptor (12) mounted on the endoscope/laparoscope (10) is located within the main chassis (31) and is compressed to the main chassis (31) by means of the upward force coming from the movable flange (32). As it can be seen in Figure 6A, there is an upper stopper pin (44) on top of the pressing arm (35) and a lower stopper pin (45) under it. The upper stopper pin (44) limits the upward motion of the pressing arm (35) which is because of the driving force of the return spring (40). The lower stopper pin (45) limits the downward motion of the pressing arm (35) which is because of the downward force applied by the surgeon to the pressing arm (35). In the case when pressing arm (35) is closer to the upper stopper pin (44) (under the effect of the return spring (40)), the fixed intermediate adaptor (12) is in a locked condition within the main chassis (31) since it is under the effect of the return spring (40). The return spring (40) pushes the pressing arm (35) and thus the compression flange (38) and the movable flange (32) to which the compression flange (38) is connected by the connecting pins (33) in the upward direction. In this case, the movable flange (32) fastens the fixed intermediate adaptor (12) within the chassis (31) by means of compression forces. In this case, the endoscope/laparoscope (10) mounted in the fixed intermediate adaptor (12) cannot perform any relative motion with respect to the chassis (31). When the surgeon applies downward force on the pressing arm (35), in case the pressing arm (35) is between the stopper pins (44, 45), the endoscope/laparoscope (10) can only perform a rotation motion around the axis of the telescope (15). However, it is not possible to move it away from the chassis (31). In Figures 7, 8A, 8B and 8C, this situation is described. When the surgeon pushes on the pressing arm (35) to keep it in a semi-pressed condition (in a manner such that the press arm (35) is between the two stopper pins (44, 45)), the pushing force applied by the return spring (40) on the movable flange (32) is avoided and the fixed intermediate adaptor (12) can be rotated within the chassis (31) in an easily. However, the fixed intermediate adaptor (12) cannot be moved away from the chassis (31) due to the geometrical limitations and thus, the rotational motion occurs within a shape-closed revolute joint. In Figure 9, the view of the endoscope/laparoscope (10) when it is free to move away from the easy attach-detach mechanism (1) is given. In Figure 10A, a view describing the same situation is presented, in 10 B a sectional view is given and in 10C a detail view is given. Accordingly, when the surgeon presses on the pressing arm (35) until it reaches the lower stopper pin (45), the compression flange (38) to which the pressing arm (35) is in contact with, realizes a downward motion, thus the compression flange (38) pulls the movable flange (32) that it is in connection in the downward direction along the connecting pins (33). In this case, the intermediate adaptor (12) is released from the main chassis (31) due to the downward motion of the movable flange (32). When the adaptor (12) is released, it is moved away from the locking mechanism (30) by means of being pushed by the barrier pin (41) that it is in contact with. The barrier pin (41) pushes away the intermediate adaptor (12) by means of the force originated from the pre-compressed compression spring (42).

## Claims

1. An easy attach-detach mechanism (1) which allows for holding an endoscope/laparoscope (10) which is a camera system used in minimally invasive surgical systems by means of a robot or a passive holder and when necessary allows for detaching the endoscope/laparoscope (10) easily from, and attaching the endoscope/laparoscope (10) to the robot or the holder, and rotating the endoscope/laparoscope (10) around its telescope with a limited angle, **characterized in that** it further comprises:
- a fragmental fixed intermediate adaptor (12) which can be mounted on the external surface of the endoscope/laparoscope (10) that can be attached to a main chassis (31) via a linear locking mechanism (30),
- a pressing arm (35) configured to transfer downward forces applied by a surgeon to a compression flange (38),
- a movable pot (32) configured to engage with the fragmental fixed intermediate adaptor (12) and configured to prevent its motion relative to the main chassis (31) therein when the pressing arm (35) is not pressed and then allows the fragmental fixed intermediate adaptor (12) to rotate about the telescope axis when the pressing arm (35) is pressed to its half way motion, wherein the compression flange (38) is configured to transmit up and down forces transferred from the pressing arm (35) to the movable pot (32) by means of connecting pins (33),
- a return spring (40) which is attached to the main chassis (31) on one end and is attached to the pressing arm (35) on the other end and is configured to apply upward pulling force to the pressing arm;
- connecting pins (33) that are mounted on the compression flange (38) via set-screws (37) configured to connect the compression flange (38) to the movable pot (32),
- a barrier pin (41) configured to push away the fragmental fixed intermediate adaptor (12) and thus the endoscope/laparoscope (10) from the main chassis (31) as the movable pot (32) moves in the downward direction when the pressing arm (35) is pressed until it reaches a lower stopper pin (45),
- a compression spring (42) which is located behind said barrier pin (41) and inside the main chassis (31).

2. An endoscope/laparoscope easy attach-detach mechanism (1) according to claim 1, **characterized in that** it comprises a plurality of connecting pin/pins (33) which are located between the compression flange (38) and the movable pot (32) and is configured to transmit the motion coming from the compression flange (38) to the movable pot (32).

3. An endoscope/laparoscope easy attach-detach mechanism (1) according to claim 1, **characterized in that** it comprises driving springs (39) as many as the connecting pins (33) which are located on the connecting pins between the compression flange (38) and the movable pot (32) and are configured to push the movable pot (32) in the upward direction.

4. An endoscope/laparoscope easy attach-detach mechanism (1) according to claim 1, **characterized in that** it comprises an upper stopper pin (44) located on the main chassis (31) configured to limit the upward movement of the pressing arm (35) by means of the drive coming from the return spring (40).

5. An endoscope/laparoscope easy attach-detach mechanism (1) according to claim 1, **characterized in that** it comprises a lower stopper pin (45) located on the main chassis (31) configured to limit the downward movement of the pressing arm (35) as a result of the pressing force originating from the surgeon.

6. An endoscope/laparoscope easy attach-detach mechanism (1) according to claim 1, **characterized in that** it comprises an adaptor centering pin (11) which acts for centering during the assembly of the two parts of the fragmental fixed intermediate adaptor (12) on the endoscope/laparoscope (10).

7. An endoscope/laparoscope easy attach-detach mechanism (1) according to claim 1, **characterized in that** it comprises adjusting screws (14) configured to fasten the fixed intermediate adaptors (12) to the endoscope/laparoscope (10).

8. An endoscope/laparoscope easy attach-detach mechanism (1) according to claim 1, **characterized in that** it comprises driving parts (13) which are located on the inner surface of the adaptor (12) in order to limit the relative movement of the endoscope/laparoscope (10) inside the fragmental fixed intermediate adaptor (12).

9. An endoscope/laparoscope easy attach-detach mechanism (1) according to claim 1, **characterized in that** it comprises a mounting interface (20) which is mounted by means of the manually opened/closed fastener (21) after being centered by means of the centering pins (23) to the chassis (31) of the locking mechanism (30).

10. An endoscope/laparoscope easy attach-detach mechanism (1) according to claim 1, **characterized in that** it comprises an angular position sensor (24) which is assembled to the mounting interface (20) by means of the angular position sensor connecting screw (22), is engaged with the angular position sensor magnet (36) located on the main chassis (31) and is configured to measure the angular position of the pressing arm (35).

11. An endoscope/laparoscope easy attach-detach mechanism (1) according to claim 1, **characterized in that** it comprises a bearing (34) which allows the pressing arm (35) to be mounted on and to rotate relative to the main chassis (31).

12. An endoscope/laparoscope easy attach-detach mechanism (1) according to claim 1, **characterized in that** it comprises a compression spring adjusting screw (43) configured to adjust the pushing force of the compression spring (42) located behind the barrier pin (41).

## Patentansprüche

1. Einfacher Anbringungs-/Ablösemechanismus (1), der es ermöglicht, ein Endoskop/Laparoskop (10), das ein Kamerasystem ist, das in minimalinvasiven chirurgischen Systemen verwendet wird, mittels eines Roboters oder einer passiven Halterung zu halten, und der es, wenn nötig, ermöglicht, das Endoskop/Laparoskop (10) leicht von dem Roboter oder der Halterung zu lösen und das Endoskop/Laparoskop (10) daran anzubringen, und das Endoskop/Laparoskop (10) um sein Teleskop mit einem begrenzten Winkel zu drehen, **dadurch gekennzeichnet, dass** er ferner umfasst:
- einen fragmentarischen, festen Zwischenadapter (12), der an der Außenfläche des Endoskops/Laparoskops (10) montiert werden kann, der über einen linearen Verriegelungsmechanismus (30) an einem Hauptchassis (31) angebracht werden kann,
- einen Druckarm (35), der so konfiguriert ist, dass er von einem Chirurgen ausgeübte Abwärtskräfte auf einen Druckflansch (38) überträgt,
- einen beweglichen Topf (32), der so konfiguriert ist, dass er in den fragmentarischen, festen Zwischenadapter (12) eingreift und so konfiguriert ist, dass er dessen Bewegung relativ zum Hauptchassis (31) darin verhindert
wenn der Druckarm (35) nicht gedrückt wird, und es dann dem fragmentarischen, festen Zwischenadapter (12) ermöglicht, sich um die Teleskopachse zu drehen, wenn der Druckarm (35) in seine Halbwertsbewegung gedrückt wird,
wobei der Druckflansch (38) so konfiguriert ist, dass er die vom Druckarm (35) auf den beweglichen Topf (32) übertragenen Auf- und Abwärtskräfte mittels Verbindungsstiften (33) überträgt,
- eine Rückstellfeder (40), die an einem Ende am Hauptchassis (31) und am anderen Ende am Druckarm (35) angebracht ist und so konfiguriert ist, dass sie eine nach oben gerichtete Zugkraft auf den Druckarm ausübt;
- Verbindungsstifte (33), die über Gewindestifte (37) am Druckflansch (38) montiert sind, die so konfiguriert sind, dass sie den Druckflansch (38) mit dem beweglichen Topf (32) verbinden,
- einen Sperrstift (41), der so konfiguriert ist, dass er den fragmentarischen, festen Zwischenadapter (12) und somit das Endoskop/Laparoskop (10) vom Hauptchassis (31) wegdrückt, wenn sich der bewegliche Topf (32) in die Abwärtsrichtung bewegt, wenn der Druckarm (35) gedrückt wird, bis er einen unteren Anschlagstift (45) erreicht,
- eine Druckfeder (42), die sich hinter dem Sperrstift (41) und innerhalb des Hauptchassis (31) befindet.

2. Einfacher Anbringungs-/Ablösemechanismus (1) für das Endoskop/Laparoskop nach Anspruch 1, **dadurch gekennzeichnet, dass** er mehrere Verbindungsstifte (33) umfasst, die zwischen dem Druckflansch (38) und dem beweglichen Topf (32) angeordnet sind und so konfiguriert sind, dass sie die vom Druckflansch (38) kommende Bewegung auf den beweglichen Topf (32) übertragen.

3. Einfacher Anbringungs-/Ablösemechanismus (1) für das Endoskop/Laparoskop nach Anspruch 1, **dadurch gekennzeichnet, dass** er Antriebsfedern (39) in gleicher Anzahl wie die Verbindungsstifte (33) umfasst, die auf den Verbindungsstiften zwischen dem Druckflansch (38) und dem beweglichen Topf (32) angeordnet und so konfiguriert sind, dass sie den beweglichen Topf (32) in die Aufwärtsrichtung drücken.

4. Einfacher Anbringungs-/Ablösemechanismus (1) für das Endoskop/Laparoskop nach Anspruch 1, **dadurch gekennzeichnet, dass** er einen oberen Anschlagstift (44) umfasst, der sich auf dem Hauptchassis (31) befindet und so konfiguriert ist, dass er die Aufwärtsbewegung des Druckarms (35) mit Hilfe des von der Rückstellfeder (40) kommenden Antriebs begrenzt.

5. Einfacher Anbringungs-/Ablösemechanismus (1) für das Endoskop/Laparoskop nach Anspruch 1, **dadurch gekennzeichnet, dass** er einen unteren Anschlagstift (45) umfasst, der sich am Hauptchassis (31) befindet und so konfiguriert ist, dass er die Abwärtsbewegung des Druckarms (35) infolge der vom Chirurgen ausgehenden Druckkraft begrenzt.

6. Einfacher Anbringungs-/Ablösemechanismus (1) für das Endoskop/Laparoskop nach Anspruch 1, **dadurch gekennzeichnet, dass** er einen Adapterzentrierstift (11) umfasst, der zur Zentrierung während der Montage der beiden Teile der fragmentarischen, festen Zwischenadapter (12) am Endoskop/Laparoskop (10) dient.

7. Einfacher Anbringungs-/Ablösemechanismus (1) für das Endoskop/Laparoskop nach Anspruch 1, **dadurch gekennzeichnet, dass** er Stellschrauben (14) umfasst, die zur Befestigung der festen Zwischenadapter (12) am Endoskop/Laparoskop (10) konfiguriert sind.

8. Einfacher Anbringungs-/Ablösemechanismus (1) für das Endoskop/Laparoskop nach Anspruch 1, **dadurch gekennzeichnet, dass** er Antriebsteile (13) umfasst, die sich auf der Innenfläche des Adapters (12) befinden, um die relative Bewegung des Endoskops/Laparoskops (10) im Inneren des fragmentarischen, festen Zwischenadapters (12) zu begrenzen.

9. Einfacher Anbringungs-/Ablösemechanismus (1) für das Endoskop/Laparoskop nach Anspruch 1, **dadurch gekennzeichnet, dass** er eine Montageschnittstelle (20) umfasst, die mittels des manuell zu öffnenden/schließenden Verschlusses (21) montiert wird, nachdem sie mittels der Zentrierstifte (23) auf dem Chassis (31) des Verriegelungsmechanismus (30) zentriert wurde.

10. Einfacher Anbringungs-/Ablösemechanismus (1) für das Endoskop/Laparoskop nach Anspruch 1, **dadurch gekennzeichnet, dass** er einen Winkelpositionssensor (24) umfasst, der mittels der Verbindungsschraube (22) des Winkelpositionssensors an der Montageschnittstelle (20) montiert ist, mit dem Magneten (36) des Winkelpositionssensors in Eingriff steht, der sich auf dem Hauptchassis (31) befindet, und so konfiguriert ist, dass er die Winkelposition des Druckarms (35) misst.

11. Einfacher Anbringungs-/Ablösemechanismus (1) für das Endoskop/Laparoskop nach Anspruch 1, **dadurch gekennzeichnet, dass** er ein Lager (34) umfasst, das es ermöglicht, den Druckarm (35) auf dem Hauptchassis (31) zu montieren und relativ zu diesem zu drehen.

12. Einfacher Anbringungs-/Ablösemechanismus (1) Einstellstange das Endoskop/Laparoskop nach Anspruch 1, **dadurch gekennzeichnet, dass** er eine Stellschraube (43) für die Druckfeder umfasst, die so konfiguriert ist, dass sie die Druckkraft der hinter dem Sperrstift (41) angeordneten Druckfeder (42) einstellt.

## Revendications

1. Mécanisme (1) d'attache et de retrait facile qui permet de tenir un endoscope/laparoscope (10), qui est un système de caméra utilisé dans les systèmes chirurgicaux peu invasifs, au moyen d'un robot ou d'un support passif et, si nécessaire, de retirer facilement l'endoscope/laparoscope (10) du robot ou au support, et de fixer l'endoscope/laparoscope (10) à celle-ci, et de faire tourner l'endoscope/laparoscope (10) autour de son télescope avec un angle limité, **caractérisé en ce qu'**il comprend en outre :
- un adaptateur intermédiaire fixe fragmentaire (12) qui peut être monté sur la surface externe de l'endoscope/laparoscope (10) et qui peut être fixé à un châssis principal (31) par l'intermédiaire d'un mécanisme de verrouillage linéaire (30),
- un bras de pression (35) configuré pour transférer les forces vers le bas appliquées par un chirurgien à une bride de compression (38),
- un pot mobile (32) configuré pour s'engager avec l'adaptateur intermédiaire fixe fragmentaire (12) et configuré pour empêcher son mouvement par rapport au châssis principal (31) lorsque le bras de pression (35) n'est pas pressé et permet alors à l'adaptateur intermédiaire fixe fragmentaire (12) de tourner autour de l'axe du télescope lorsque le bras de pression (35) est pressé à micourse,
dans lequel la bride de compression (38) est configurée pour transmettre les forces de haut en bas transférées du bras de pression (35) au pot mobile (32) au moyen de goupilles de connexion (33),
- un ressort de rappel (40) fixé au châssis principal (31) à une extrémité et au bras de pression (35) à l'autre extrémité, configuré pour appliquer une force de traction vers le haut au bras de pression ;
- des goupilles de connexion (33) qui sont montées sur la bride de compression (38) par l'intermédiaire de vis sans tête (37) configurées pour connecter la bride de compression (38) au pot mobile (32),
- une goupille de barrière (41) configurée pour repousser l'adaptateur intermédiaire fixe fragmentaire (12) et donc l'endoscope/laparoscope (10) du châssis principal (31) lorsque le pot mobile (32) se déplace vers le bas lorsque le bras de pression (35) est pressé jusqu'à ce qu'il atteigne une goupille d'arrêt inférieure (45),
- un ressort de compression (42) qui est situé derrière ladite goupille de barrière (41) et à l'intérieur du châssis principal (31).

2. Mécanisme (1) d'attache et de retrait facile pour endoscope/laparoscope selon la revendication 1, **caractérisé en ce qu'**il comprend une pluralité de goupilles de connexion (33) qui sont situées entre la bride de compression (38) et le pot mobile (32) et est configuré pour transmettre le mouvement provenant de la bride de compression (38) au pot mobile (32).

3. Mécanisme (1) d'attache et de retrait facile pour endoscope/laparoscope selon la revendication 1, **caractérisé en ce qu'**il comprend des ressorts d'entraînement (39) autant que les goupilles de connexion (33) qui sont situés sur les goupilles de connexion entre la bride de compression (38) et le pot mobile (32) et sont configurés pour pousser le pot mobile (32) dans la direction ascendante.

4. Mécanisme (1) d'attache et de retrait facile pour endoscope/laparoscope selon la revendication 1, **caractérisé en ce qu'**il comprend une goupille d'arrêt supérieure (44) située sur le châssis principal (31) configurée pour limiter le mouvement vers le haut du bras de pression (35) au moyen de l'entraînement provenant du ressort de rappel (40).

5. Mécanisme (1) d'attache et de retrait facile pour endoscope/laparoscope selon la revendication 1, **caractérisé en ce qu'**il comprend une goupille d'arrêt inférieure (45) située sur le châssis principal (31) configurée pour limiter le mouvement vers le bas du bras de pression (35) sous l'effet de la force de pression provenant du chirurgien.

6. Mécanisme (1) d'attache et de retrait facile pour endoscope/laparoscope selon la revendication 1, **caractérisé en ce qu'**il comprend une goupille de centrage (11) de l'adaptateur qui sert à centrer pendant l'assemblage des deux parties des adaptateurs intermédiaires fixes fragmentaires (12) sur l'endoscope/laparoscope (10).

7. Mécanisme (1) d'attache et de retrait facile pour endoscope/laparoscope selon la revendication 1, **caractérisé en ce qu'**il comprend des vis de réglage (14) configurées pour fixer les adaptateurs intermédiaires fixes (12) à l'endoscope/laparoscope (10).

8. Mécanisme (1) d'attache et de retrait facile pour endoscope/laparoscope selon la revendication 1, **caractérisé en ce qu'**il comprend des pièces d'entraînement (13) situées sur la surface intérieure de l'adaptateur (12) afin de limiter le mouvement relatif de l'endoscope/laparoscope (10) à l'intérieur de l'adaptateur intermédiaire fixe fragmentaire (12).

9. Mécanisme (1) d'attache et de retrait facile pour endoscope/laparoscope selon la revendication 1, **caractérisé en ce qu'**il comprend une interface de montage (20) qui est montée au moyen de la fixation à ouverture/fermeture manuelle (21) après avoir été centrée au moyen des goupilles de centrage (23) sur le châssis (31) du mécanisme de verrouillage (30).

10. Mécanisme (1) d'attache et de retrait facile pour endoscope/laparoscope selon la revendication 1, **caractérisé en ce qu'**il comprend un capteur de position angulaire (24) qui est assemblé à l'interface de montage (20) au moyen de la vis de connexion du capteur de position angulaire (22), qui s'engage avec l'aimant du capteur de position angulaire (36) situé sur le châssis principal (31) et qui est configuré pour mesurer la position angulaire du bras de pression (35).

11. Mécanisme (1) d'attache et de retrait facile pour endoscope/laparoscope selon la revendication 1, **caractérisé en ce qu'**il comprend un palier (34) qui permet au bras de pression (35) d'être monté sur le châssis principal (31) et de tourner par rapport à celui-ci.

12. Mécanisme (1) d'attache et de retrait facile pour endoscope/laparoscope selon la revendication 1, **caractérisé en ce qu'**il comprend une vis de réglage (43) du ressort de compression configurée pour ajuster la force de poussée du ressort de compression (42) situé derrière la goupille de barrière (41).
